# EUROPEAN PATENT SPECIFICATION

(11) **EP 0 835 846 B1**
(45) Date of publication and mention of the grant of the patent: **03.04.2002**
(21) Application number: 97117291.1
(22) Date of filing: 07.10.1997
(51) Int. Cl.: C02F 3/30

(54) **A method of bio-aeration of waste water and a device for carrying out the method**
Verfarhen zur Biobelüftung von Abwasser und Vorrichtung zur Durchführung des Verfahrens
Procédé pour l'aération biologique de l'eau usée et dispositif pour la mise en oeuvre du procédé

(30) Priority: 11.10.1996 SK 131396
(43) Date of publication of application: 15.04.1998
(73) Proprietor: Ekoprogres v.d., 911 06 Trencin (SK)
(72) Inventor: Stefan, Mico, 91106 Trencin (SK)
(74) Representative: Bergmeier, Werner, Dipl.-Ing.

(56) References cited:
- EP-A- 0 014 394
- DE-A- 19 547 364
- US-A- 4 522 722
- US-A- 5 514 278

## Description

The invention relates to a method of bio-aeration of waste water and a device for carrying out the method, with the application of an anaerobic degree and an aerobic degree, in particular of waste water affected by medium to heavy contamination by organic substances.

The up to now known method of waste water purification with the removal of waste water heavily contaminated by organic substances is disclosed in the published patent application CS 9 000 076.

According to this method, the functional polyculture of activated sediment is separated from the activating mixture produced in the aeorobic zone from the partly purified waste water. The waste water is separately treated by nitrification, then mixed with the previously separated functional polyculture, and the mixture is subject to denitrification in an anoxic reactor, and to aeration, in an oxic reactor. The separated functional polyculture is recirculated back into the anaerobic reactor, and the purified waste water is led into a recipient. The purification process is relatively lengthy, and although it uses anaerobic steps, it does so only for the anaerobic treatment of the functional polyculture separated from the activated sediment.

An up to now known device for bio-aeration of waste water containing carbonaceous and nitrogeneous substances disclosed in the CS author's certificate 228 208 consists of two containers. In one of them is a separation space situated over a denitrification space, the other contains the activation space. The separation space is separated from the denitrification space by a slanting partition, and is connected with this space by a sediment outlet.

The drawback of this solution consists in the use of two containers without aerobie purification. If these two containers are situated separately, the device takes up a considerable space.

Another known device for the nitrification and denitrification of organic substances in waste water, disclosed in the author's certificate CS A'0 248 616 features the activation space divided by a partition into an aerated and a non-aerated part. The partition reaches partially or totally above the water level, and creates a slot at the bottom of the container.

In its design, the device is very simple but it contains no separation section and no anaerobic pre-purification indispensable for achieving high quality degree of purification.

The above drawbacks are to a considerable extent eliminated by the method of bio-aeration of waste water using the two-degree anaerobic-aerobic method of purification, in particular of waste water with medium to heavy contamination by organic substances, with the carrying out of complete anaerobic purification with the production of biogas in the first stage, continually followed by the stage of the complete aerobic purification in the second degree whose principle consists in that acidification treated raw waste water is first led into the anaerobic degree under constant mixing with the recirculated condensed sediment of the anaerobic degree together with the recirculated condensed sediment of the. aerobic degree with the following separation of the biogas in process of formation from the activated sediment and the pre-purified water which latter flows into the. aerobic degree where, in a regeneration zone and under intense aeration, it is mixed with the recirculated condensed activated sediment, and then flows through the anoxic zone into the oxic zone, with the following separation of the purified water from the activated sediment whose one part is recirculated into the generation zone and whose the other part is mixed with the acidification treated raw water before entering the anaerobic degree.

The advantages of this method consist in high utility parameters and in very high purification output, the purification process itself taking place under usual physical conditions. Besides, the method combines the advantages of the anaerobic and aerobic purification with a very low production of superfluous sediment and with the production of biogas as a highly valuable fuel.

The device for carrying out the method preferably comprises an outer container, circle- or angular-shaped in plan view and fitted with nozzles at its bottom, an inlet for raw waste water and an outlet for purified water, and its principle consists in that the container is separated by a partition into an. anaerobic degree and into an aerobic degree consisting of a regeneration zone, of an anoxic zone, and of an oxic zone with a separation. The lower part of the container, serving as the anaerobic degree, is fitted with an inlet of the raw waste water equipped with a blender connected to an inlet of condensed recirculated anaerobic sediment and to an inlet of aerobic condensed superfluous sediment. Provided in the upper part of the. anaerobic degree is a three-phase separator fitted with a gas outlet, a sediment outlet, and a pre-purified water outlet. In the upper part of the aerobic degree is situated a separator with a positioned aperture and outlet of the purified water, and the lower part of the aerobic degree holds the regeneration zone with an outlet aperture of the pre-purified water and an inlet aperture of the recirculated activated sediment.

The device has a simple modular design with minimum outer space requirements because all the purification degrees of waste water are situated in one appropriately separated space. The adaptability of the device to various waste water composition in combination with the intensity of the purification process completely meets the economic requirements on the quality of the purified water.

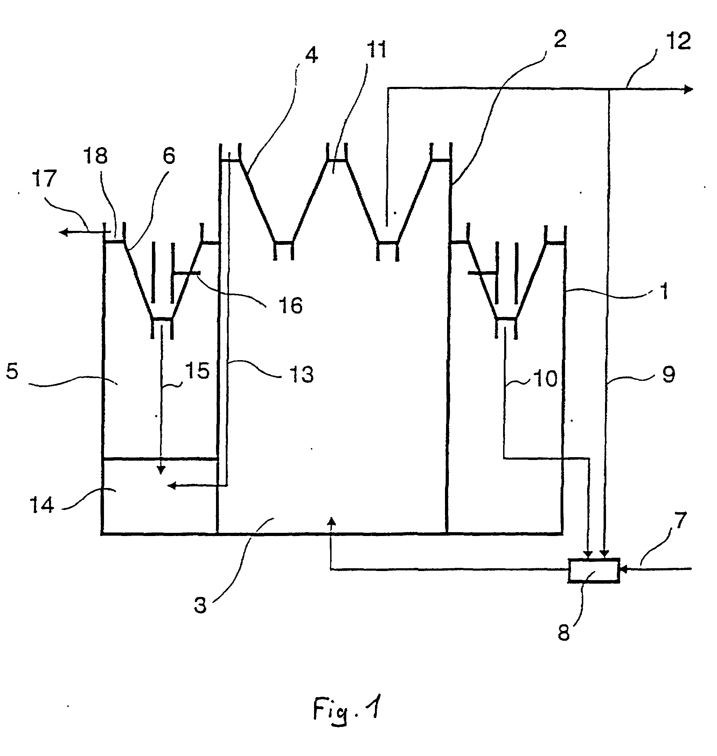

The invention will be now explained in more details with reference to the accompanying drawing in which Fig. 1 shows the device according to the invention in a longitudinal axis section.

The method of bio-aeration of waste water consists in a two-degree anaerobic-aerobic purification process in which in its first degree there takes place the complete anaerobic purification accompanied by the biogas production continually followed by the second degree with the complete final aerobic purification. The raw waste water adapted by acidification is first led into the first anaerobic degree under constant mixing with the recirculated condensed sediment of the anaerobic degree together with the recirculated condensed sediment of the. aerobic degree, for instance in a ratio of 1:1:0.01 that can vary depending on the contaminating substance of the coming water. The laminar mixing of the mixture thus created keeps the activated sediment up with continual separation of the biogas in process of production from the activated sediment and pre-purified water flowing into the aerobic degree. In the regeneration zone, it is mixed with the recirculated condensed activated sediment and flows under intense aeration into the anoxic zone of the aerobic degree and further into the oxic zone of the same degree. In the oxic zone there goes on continual separation of the purified water from the activated sediment whose one part recirculates into the regeneration zone and whose the other part is mixed with the raw water adapted by acidification before entering the. anaerobic degree, for instance in a ratio of 3:1.

The device for carrying out the method according to Fig. 1 consists of an outer container 1, circular- or angular-shaped in plan view, and having its bottom equipped with not represented nozzles. The container 1 is separated by a partition 2 into a anaerobic degree 3, and into an aerobic degree 5 consisting of a regeneration zone 14 and of a not represented anoxic zone and oxic zone. The lower part of the container 1 contains a supply 7 of the raw waste water fitted with a blender 8 connected with a supply 9 of the condensed recirculated anaerobic sediment and with a supply 10 of the superfluous aerobic condensed sediment. Situated in the upper part of the anaerobic degree are a three-phase separator 4 fitted with a biogas outlet 11, a sediment outlet 12 and an outlet 13 for pre-purified water. Situated in the upper part of the. aerobic degree is a separator 6 featuring an aperture 16 and a collecting trough 18 fitted with an outlet 17 for the purified water. The lower part of the aerobic degree 5 contains the regeneration zone 14 connected with the outlet 13 for the pre-purified water and an inlet 15 for the recirculated activated sediment.

The procedure of purification of waste water according to the invention is clearly shown in the figure. In the blender 8, raw waste water is mixed with the recirculated condensed sediment from the anaerobic degree 3 together with the recirculated condensed sediment from the aerobic degree 5. The mixture thus created flows through the inlet nozzles into the lower part of the anaerobic degree 3 thus ensuring the laminar mixing of the mixture in the anaerobic degree 3 and thus permanently keeping the activated sediment up, with continual separation of the biogas in process of formation from the activated sediment and from the pre-purified water that flows through the outlet 13 of pre-purified water into the aerobic degree 5. The biogas is let out by the biogas outlet 11. In the regeneration zone 14 of the. aerobic degree 5, the pre-purified water is mixed (blended) with the recirculated condensed activated sediment and flows under intense aeration into the anoxic zone of the. aerobic degree 5 and then into the oxic zone of the same degree. In the oxic zone, the purified water is separated from the activated sediment whose one part recirculates through the inlet 15 of the recirculated activated sediment into the regeneration zone 14 and whose the other part passes through the inlet 10 of the superfluous condensed aerobic sediment into the blender 8 where it is mixed with the raw water treated by acidification, before the entry into the anaerobic degree 3, for instance in a ratio of 3 to 1. Under intense aeration, the mixture passes into the anoxic zone of the aerobic degree 5 where the nitrogeneous contamination is degradated. The mixture flows then first into the oxic zone of the aerobic degree 5 where it is aerated by nozzles provided in the bottom of the container 1 and then into the separator 6 in which the separation of the activated sediment takes place. One part of it is recirculated by the inlet 15 of the recirculated activated sediment back into the regeneration zone 14 of the aerobic degree 5, and the other part of it is led into the blender 8 situated before the anaerobic degree 3: The three-phase separator 4 separates the biogas in process of formation, let out by the biogas outlet 11, from the activated sediment, let out by the sediment outlet 12, and from the pre-purified water, let out by the outlet 13. The pre-purified water flows into the aerobic degree 5 through the regeneration zone 14 of the anaerobic degree 5 receiving under intense aeration the activated sediment through the inlet 15. The created activation mixture flows into a not represented anoxic zone and oxic zone and from there through the aperture 16 into the separator 6 where the sediment is separated from the purified water led by the collecting through into the outlet 17 for purified water.

The device has a wide application in the waste water treatment plants. By its design and efficiency, it meets both the ecological and economic demands on the quality of the purified water.

## Claims

1. A method of bio-aeration of waste water using the two-degree anaerobic-aerobic method of purification, in particular of waste water with medium to heavy contamination by organic substances, with the carrying out of complete anaerobic purification with the production of biogas in the firstdegree, continually followed by the complete aerobic purification in the second degree **characterized in that**, raw waste water treated by acidification is first led into the anaerobic degree (3) under constant mixing with the recirculated condensed sediment of the anaerobic degree (3) together with the recirculated condensed sediment of the aerobic degree (5) with the following separation of the biogas in process of formation from the activated sediment and the pre-purified water which latter flows into the aerobic degree (5) where, in a regeneration zone (14) and under intense aeration, it is mixed with the recirculated condensed activated sediment, and then flows through the anoxic zone into the oxic zone, with the following separation of the purified water from the activated sediment whose one part is recirculated into the generation zone (14) and whose the other part is mixed with the acidification treated raw water before entering the anaerobic degree (3).

2. A device for carrying out the method as claimed in Claim 1, consisting of an outer container, circular- or angular-shaped in plan view and fitted with nozzles at its bottom, of a supply (7) of raw waste water, and of an outlet for the purified water, **characterized in that** the container (1) is separated by a partition (2) into an anaerobic degree (3) and into an aerobic degree (5) consisting of a regeneration zone, of an anoxic zone, and of an oxic zone with a separation in which the lower part of the container (1), serving as the anaerobic degree (3), is fitted with a supply (7) for the raw waste water equipped with a blender (8) connected to an inlet (supply) (9) of condensed recirculated anaerobic sediment and to an inlet (supply) (10) of aerobic condensed superfluous sediment, in which in the upper part of the anaerobic degree (3) there is provided a three-phase separator (4) fitted with a biogas outlet (11), with a sediment outlet (12), and with an outlet (13) for prepurified water, and in which in the upper part of the aerobic degree (5) there is provided a separator (6) with a positioned aperture (16) and with a collecting trough (17) for the purified water while the lower part of the . aerobic degree houses the regeneration zone (14) with an inlet for the pre-purified water and with the inlet (15) for the recirculated activated sediment.

## Patentansprüche

1. Verfahren zur biologischen Abfallwasserreinigung im Zweistufen-Anaerob-Aerobverfahren, besonders für Abfallwasser mit mittlerer bis hoher Kontaminierung durch organische Stoffe, mit Durchführung kompletter anaerobischer Reinigung in der ersten Stufe, fließend gefolgt durch die zweite Stufe mit vollständiger aerobischer Reinigung, **dadurch gekennzeichnet, daß** das durch Azidifikation aufbereitete rohe Abfallwasser zuerst unter ständigem Rühren mit rezirkuliertem verdichtetem Schlamm von der Anaerobstufe (3) gemeinsam mit rezirkuliertem verdichtetem Schlamm von der Aerobstufe (5) in die Anaerobstufe zugeführt wird mit der nachfolgenden Separation des entstehenden Biogases vom aktivierten Schlamm und vom vorgereinigten Wasser, das in die Aerobstufe (5) fließt, wo es sich in einer Regenerierungszone (14) unter intensivem Durchlüften mit dem rezirkulierten verdichteten Schlamm vermischt und weiter durch die anoxische Zone in die oxische Zone fließt mit anschließender Separierung des gereinigten Wassers vom aktivierten Schlamm, dessen ein Teil in die Regenerierungszone (14) rezirkuliert und dessen der andere Teil vor dem Eingang in die Anaerobstufe (3) mit dem durch Azidifikation aufbereiteten rohen Abfallwasser vermischt wird.

2. Vorrichtung zur Durchführung des Verfahrens nach Anspruch 1, bestehend aus einem äußeren Behälter (1) mit rundem oder eckigem Grundriß, dessen Boden mit Düsen versehen ist, aus einer Zuleitung für rohes Abfallwasser und einer Ableitung für gereinigtes Wasser, **dadurch gekennzeichnet, daß** der Behälter (1) durch eine Teilungswand (2) in eine Anaerobstufe (3) und eine Aerobstufe (5) eingeteilt ist, bestehend aus einer Regenerierungszone, einer anoxischen Zone und einer oxischen Zone mit der Separierung, wobei im unteren, die Anaerobstufe (3) bildenden Teil des Behälters die Zuleitung (7) des rohen Abfallwassers vorgesehen ist, die mit einem Vermischer (8) versehen ist, in den eine Zuleitung (9) des verdichteten rezirkulierten anaeroben Schlamms und eine Zuleitung (10) des überflüssigen verdichteten aeroben Schlamms mündet, wobei im oberen Teil der Anaerobstufe (3) ein mit einer Biogasableitung (11), einer Schlammableitung (12) und einer Ableitung (13) für vorgereinigtes Wasser versehener Dreiphasen-Abscheider (4) untergebracht ist, wobei im oberen Teil der Aerobstufe (5) ein Separator (6) mit einer positionnierten Öffnung (16) und mit einer Sammelrinne (17) für das gereinigte Wasser untergebracht ist, und wobei im unteren Teil der Aerobstufe (5) die Regenerierungszone (14) untergebracht ist, in die eine *Zuleitung* (13) des vorgereinigten Wassers und eine Zuleitung (15) des rezirkulierten aktivierten Schlamms münden.

## Revendications

1. Méthode d'épuration des eaux industrielles par un procédé d'épuration à deux degrés aérobique-anaérobique, surtout des eaux de contamination moyenne à élevée par substances organiques, avec la réalisation d'épuration complète anaérobique accompagnée de la formation du gaz de fumier dans la première phase suivie en continu de la seconde phase produisant l'épuration complète aérobique, **caractérisée en ce que** l'eau industrielle brute modifiée par acidification est conduite d'abord au degré anaérobique (3) sous mélange constant avec le sédiment condensé récirculé/recyclé du degré anaérobique (3) ensemble avec le sédiment condensé récirculé/recyclé du degré anaérobique (5), avec la suivante séparation du gaz de fumier et du sédiment activé et de l'eau préépurée qui coule vers et dans le degré aérobique (5) où il se mélange dans une zone de régénération (14) avec le sédiment condensé activé et en même temps subit une aération intense et passe ensuite par la zone anoxique dans la zone oxique avec la suivante séparation de l'eau épurée du sédiment activé dont une part récircule dans la zone de régénération (14) et dont l'autre part se mélange avec l'eau brute modifiée par acidification avant d'entrer dans le degré anaérobique (3).

2. Dispositif pour exécuter la méthode suivant la revendication 1 qui se compose d' un récipient extérieur à vue en plan ronde ou angulaire dont le fond est muni de buses, d'une conduite d'amenée (7) de l'eau industrielle brute e d'une conduite de sortie de l'eau épurée, **caractérisé en ce que** le récipient (1) est divisé par une paroi de séparation (2) en un degré anaérobique (3) et en un degré aérobique (5) qui se compose d'une zone de régénération, d'une zone anoxique et d'une zone oxique avec séparation, la partie inférieure du récipient (1) qui constitue le degré anaérobique (3) étant munie d'une conduite d'amenée (7) de l'eau industrielle brute équipée d'un mélangeur (8) dans lequel sont introduits le bout d'une conduite d'amenée (9) du sédiment anaérobique récirculé et le bout d'une conduite d'amenée (10) du sédiment condensé aérobique superflu tandis que la partie supérieure du degré anaérobique (3) comprend un séparateur à trois phases (4) muni d'une conduite de sortie (11) du gaz de fumier, d'une conduite de sortie (12) du sédiment, et d'une conduite de sortie (13) de l'eau préépurée et tandis que la partie supérieure du degré aérobique (5) comprend un séparateur (6) avec un orifice (16) situé et avec une auge de collection de l'eau épurée et tandis que dans la partie inférieure du degré aérobique (5) est située la zone de régénération (14) dans laquelle sont introduits par leurs bouts une conduite d'amenée (13) de l'eau préépurée et une conduite d'amenée (15) du sédiment récirculé activé.
